# EUROPEAN PATENT APPLICATION

(11) **EP 4 420 700 A1**
(43) Date of publication of application: **28.08.2024**
(21) Application number: 23305254.7
(22) Date of filing: 24.02.2023
(51) Int. Cl.: A61M 5/32, A61M 5/31, A61M 5/20, A61M 5/28

(54) **DRUG DELIVERY DEVICE COMPRISING A CAP**

(71) Applicant: Becton, Dickinson and Company, Franklin Lakes, NJ 07417-1880 (US)
(72) Inventor: BESSON, Nicolas, 38650 Treffort (FR); PLOUVIER, Adrien, 38400 SAINT MARTIN D'HERES (FR); ALVAIN, Olivier, 38180 SEYSSINS (FR)
(74) Representative: Germain Maureau

(57) **Abstract**

Provided herein is a drug delivery device including: a housing having a first end including a distal region and an adjacent proximal region, where in a first region configured to engage a clip from a cap, the distal region includes a raised plateau region relative to an adjacent region of the distal region and the proximal region includes a recess adjacent to the plateau region; and the cap including a distal end and a proximal end, where the proximal end of the cap is engageable with the first end of the housing, where the cap includes a wall defining a perimeter and the clip protruding from the wall at the proximal end of the cap, where when the housing and the cap are engaged, the wall of the cap is in contact with the plateau region of the housing, and the clip resides in the recess of the housing.

## Description

### BACKGROUND OF THE INVENTION

### Field of the Invention

The present disclosure relates generally to a drug delivery device and, in some non-limiting embodiments, to a drug delivery device comprising a cap.

### Description of Related Art

Various types of automatic injection devices have been developed to allow drug solutions and other liquid therapeutic preparations to be administered by untrained personnel or to be self-injected. Generally, these devices include a reservoir that is pre-filled with the liquid therapeutic preparation, and some type of automatic needle-injection mechanism that can be triggered by the user. Many of these devices, such as auto-injectors, are designed so that the reservoir, such as a pre-filled syringe, is assembled into the device during assembly of the device. In addition to automatically deploying the needle-injection mechanism, many drug delivery devices also automatically shield the needle after use of the device to prevent any unintended contact with the needle.

In some automatic injection devices, prior to use, a cap is included over the end deploying the needle-injection mechanism as an additional safety feature. It is desirable for the cap to be secure over the end to prevent unwanted dislodging thereof, while not being prohibitively difficult to intentionally remove by the user at the time of injection.

### SUMMARY OF THE INVENTION

Provided herein is a drug delivery device including: a housing having a first end and a second end, where the first end includes a distal region defining a perimeter of the housing and an adjacent proximal region defining the perimeter of the housing, where in a first region configured to engage a clip from a cap, the distal region includes a raised plateau region relative to an adjacent region of the distal region and the proximal region includes a recess adjacent to the plateau region; and the cap including a distal end and a proximal end, where the proximal end of the cap is engageable with the first end of the housing, where the cap includes a wall defining a perimeter and the clip protruding from the wall at the proximal end of the cap, where when the housing and the cap are engaged, the wall of the cap is in contact with the plateau region of the housing, and the clip resides in the recess of the housing.

Also provided herein is a method of manufacturing a drug delivery device, including: engaging a housing of a drug delivery device with a cap, where: the housing includes a first end and a second end, where the first end includes a distal region defining a perimeter of the housing and an adjacent proximal region defining the perimeter of the housing, where in a first region configured to engage a clip from the cap, the distal region includes a raised plateau region relative to an adjacent region of the distal region and the proximal region includes a recess adjacent to the plateau region; and the cap includes a distal end and a proximal end, where the proximal end of the cap is engageable with the first end of the housing, where the cap includes a wall defining a perimeter and the clip protruding from the wall at the proximal end of the cap, where when the housing and the cap are engaged, the wall of the cap is in contact with the plateau region of the housing, and the clip resides in the recess of the housing.

Also provided herein is a method of injecting a drug from a drug delivery device, including: disengaging a housing of a drug delivery device including a drug from a cap, where: the housing includes a first end and a second end, where the first end includes a distal region defining a perimeter of the housing and an adjacent proximal region defining the perimeter of the housing, where in a first region configured to engage a clip from the cap, the distal region includes a raised plateau region relative to an adjacent region of the distal region and the proximal region includes a recess adjacent to the plateau region; and the cap includes a distal end and a proximal end, where the proximal end of the cap is engageable with the first end of the housing, where the cap includes a wall defining a perimeter and the clip protruding from the wall at the proximal end of the cap, where when the housing and the cap are engaged, the wall of the cap is in contact with the plateau region of the housing, and the clip resides in the recess of the housing; and injecting a needle contained in the first end of the housing into a patient to inject the drug into the patient.

Other non-limiting embodiments of the invention will be set forth in the following numbered clauses:
Clause 1: A drug delivery device, comprising: a housing having a first end and a second end, wherein the first end comprises a distal region defining a perimeter of the housing and an adjacent proximal region defining the perimeter of the housing, wherein in a first region configured to engage a clip from a cap, the distal region comprises a raised plateau region relative to an adjacent region of the distal region and the proximal region comprises a recess adjacent to the plateau region; and the cap comprising a distal end and a proximal end, wherein the proximal end of the cap is engageable with the first end of the housing, wherein the cap comprises a wall defining a perimeter and the clip protruding from the wall at the proximal end of the cap, wherein when the housing and the cap are engaged, the wall of the cap is in contact with the plateau region of the housing, and the clip resides in the recess of the housing.
Clause 2: The drug delivery device of clause 1, wherein the housing comprises a plurality of first regions, each configured to engage a clip from the cap.
Clause 3: The drug delivery device of clause 1 or 2, wherein an arrangement of the clip residing in the recess of the housing defines a gap between a bottom of the clip and a base of the recess and/or defines a gap between a distal end of the clip and a distal end of the recess.
Clause 4: The drug delivery device of clause 3, wherein the distal end of the clip and the distal end of the recess form substantially the same angle relative to the plateau region.
Clause 5: The drug delivery device of clause 3 or 4, wherein the distal end of the recess is chamfered.
Clause 6: The drug delivery device of any of clauses 1-5, wherein the housing comprises a chamfered distal end adjacent a distal end of the plateau region.
Clause 7: The drug delivery device of any of clauses 1-6, wherein the adjacent region of the distal region is recessed relative to an adjacent region of the proximal region.
Clause 8: The drug delivery device of any of clauses 1-7, wherein the housing further comprises a second region configured to engage a rib from the cap, wherein in the second region, the distal region comprises a second raised plateau region relative to an adjacent region of the distal region.
Clause 9: The drug delivery device of clause 8, wherein the cap comprises the rib protruding from the wall of the cap, wherein, when the housing and the cap are engaged, the rib of the cap is in contact with the second plateau region of the housing.
Clause 10: The drug delivery device of clause 9, wherein the cap comprises a gap between a top of the rib and the proximal end of the cap such that a gap is formed between the proximal region of the housing and the wall of the cap when the housing and the cap are engaged.
Clause 11: The drug delivery device of any of clauses 8-10, wherein the housing comprises a plurality of second regions, each configured to engage a rib from the cap.
Clause 12: The drug delivery device of any of clauses 9-11, wherein the housing comprises a chamfered distal end adjacent a distal end of the second plateau region, wherein the rib comprises a distal end shaped so as to define a gap between the rib and the chamfered distal end adjacent the distal end of the second plateau region when the housing and the cap are engaged.
Clause 13: The drug delivery device of any of clauses 1-12, wherein the cap engages an outside surface of the housing.
Clause 14: The drug delivery device of any of clauses 8-13, wherein an entire surface of the plateau region and/or second plateau region are substantially parallel to the wall of the cap when the cap and the housing are engaged.
Clause 15: The drug delivery device of clause 14, wherein the wall of the cap is in contact with the plateau region of the housing across the entire surface of the plateau region, and/or the rib of the cap is in contact with the second plateau region of the housing across the entire surface of the second plateau region.
Clause 16: The drug delivery device of any of clauses 1-15, wherein the proximal end of the cap is reversibly engageable with the first end of the housing.
Clause 17: The drug delivery device of any of clauses 1-16, wherein the drug delivery device comprises an auto-injector drug delivery device.
Clause 18: The drug delivery device of any of clauses 1-17, wherein the drug delivery device comprises: the housing; a syringe comprising a barrel, a stopper, and a needle arranged at a distal end of the syringe, at least a portion of the syringe positioned within the housing; a drive assembly comprising a drive member and a plunger assembly comprising: a plunger body having a proximal end, a distal end, and a sidewall therebetween, the plunger body comprising a head at the proximal end thereof, the head defining an opening; and a plunger rod having a proximal end and a distal end, the plunger rod comprising a clip at the proximal end thereof, wherein the clip is configured to be received within the opening of the plunger body head and to limit axial displacement between the plunger rod and the plunger body, and to limit radial deflection of the plunger rod; the drive assembly configured to move the stopper within the barrel upon actuation of the drive assembly, at least a portion of the drive assembly positioned within the housing; and a needle cover having a pre-use position where the needle is positioned within the needle cover, an actuation position where the needle cover has been shifted proximally, thereby allowing the drive assembly to be actuated, and a post-use position where the needle is positioned within the needle cover.
Clause 19: A method of manufacturing a drug delivery device, such as the drug delivery device of any of clauses 1-18, comprising: engaging a housing of a drug delivery device with a cap, wherein: the housing comprises a first end and a second end, wherein the first end comprises a distal region defining a perimeter of the housing and an adjacent proximal region defining the perimeter of the housing, wherein in a first region configured to engage a clip from the cap, the distal region comprises a raised plateau region relative to an adjacent region of the distal region and the proximal region comprises a recess adjacent to the plateau region; and the cap comprises a distal end and a proximal end, wherein the proximal end of the cap is engageable with the first end of the housing, wherein the cap comprises a wall defining a perimeter and the clip protruding from the wall at the proximal end of the cap, wherein when the housing and the cap are engaged, the wall of the cap is in contact with the plateau region of the housing, and the clip resides in the recess of the housing.
Clause 20: A method of injecting a drug from a drug delivery device, such as the drug delivery device of any of clauses 1-18, comprising: disengaging a housing of a drug delivery device comprising a drug from a cap, wherein: the housing comprises a first end and a second end, wherein the first end comprises a distal region defining a perimeter of the housing and an adjacent proximal region defining the perimeter of the housing, wherein in a first region configured to engage a clip from the cap, the distal region comprises a raised plateau region relative to an adjacent region of the distal region and the proximal region comprises a recess adjacent to the plateau region; and the cap comprises a distal end and a proximal end, wherein the proximal end of the cap is engageable with the first end of the housing, wherein the cap comprises a wall defining a perimeter and the clip protruding from the wall at the proximal end of the cap, wherein when the housing and the cap are engaged, the wall of the cap is in contact with the plateau region of the housing, and the clip resides in the recess of the housing; and injecting a needle contained in the first end of the housing into a patient to inject the drug into the patient.

### BRIEF DESCRIPTION OF THE DRAWINGS

FIG. 1 is a perspective view of a drug delivery device according to non-limiting embodiments described herein;
FIG. 2 is a cross-sectional view of a drug delivery device according to non-limiting embodiments described herein;
FIG. 3 is a perspective of a drug delivery device comprising a cap according to non-limiting embodiments described herein;
FIG. 4 is a cross-sectional view of a drug delivery device engaging a cap according to non-limiting embodiments described herein;
FIG. 5 is a perspective view of a first end of a housing of a drug delivery device according to non-limiting embodiments described herein;
FIG. 6 is a perspective view of a cap for a drug delivery device according to non-limiting embodiments described herein;
FIG. 7 is a perspective view of a first end of a housing of a drug delivery device according to non-limiting embodiments described herein;
FIG. 8 is a perspective view of a rib of a cap for a drug delivery device according to non-limiting embodiments described herein; and
FIG. 9 is a cross-sectional view of a drug delivery device engaging a cap according to non-limiting embodiments described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

International Patent Application Publication Nos. WO 2020/173991, WO 2020/173992, WO 2020/173993, WO 2020/173994, and WO 2020/173995 are incorporated herein by reference in their entirety.

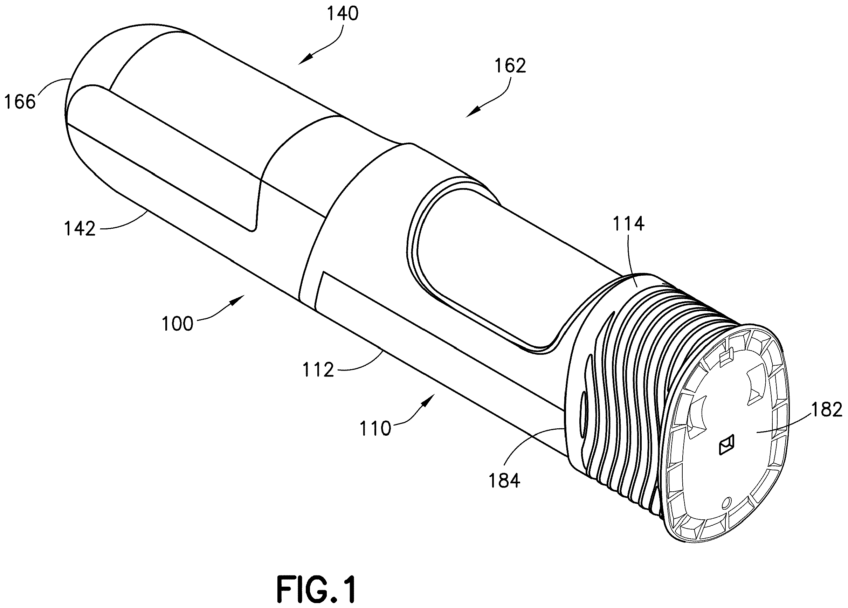

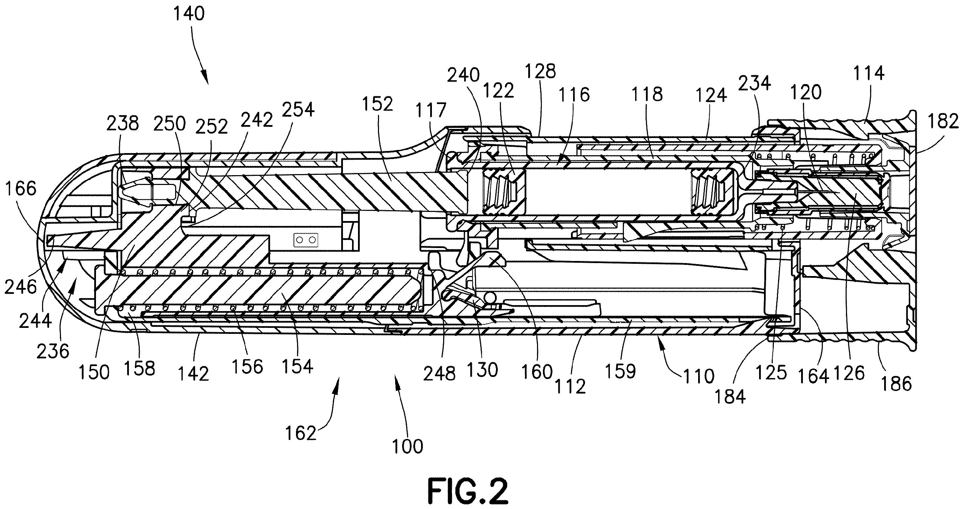

Referring to FIGS. 1 and 2, shown is a drug delivery device 100 comprising a housing 162. Drug delivery device 100 may include a first subassembly 110, a second subassembly 140, and a syringe 116. The first subassembly 110 may include lower housing shell 112, a cap 114, a syringe holder 118, a needle cover 124, and a cassette body 128. The second subassembly 140 may include a drive assembly 236 comprising a driver member 156 and a plunger assembly 244. At least a portion of the drive assembly 236 may be positioned within the housing 162. The plunger assembly 244 may include a plunger body 150 and a plunger rod 152. The plunger body 150 may have a proximal end 246, a distal end 248, and a sidewall 250 therebetween. The plunger body 150 may comprise a spring guide member 154 including the drive member 156 and a drive opening 158. The plunger body 150 may comprise a head 252 at a proximal end thereof, the head 252 defining an opening 254. Second subassembly 140 may further include a motor body 159, a lever actuation member 130, and an upper housing shell 142. The syringe 116 may be received by the syringe holder 118 and includes a barrel 117, a stopper 122, a needle 120, and a rigid needle shield (RNS) 126. The needle 120 may be arranged at a distal end 234 of the syringe 116. At least a portion of the syringe 116 may be positioned within the housing 162. Although an RNS is utilized, other suitable needle shield arrangements may be utilized. The lower housing shell 112, the cassette body 128, and the upper housing shell 142 generally form the housing 162 for receiving the various components of the drug delivery device 100, although other suitable housing arrangements may be utilized. The first subassembly 110 and the second subassembly 140 may be secured to each other during assembly by a locking clip, although other suitable arrangements may be utilized. The drug delivery device 100 may be an auto-injector, although the features described herein may be incorporated into other suitable drug delivery devices.

Drug delivery device 100 may be configured to automatically deliver a dose of medicament from the syringe 116 to a patient upon actuation of the device 100. More specifically, upon actuation of the drug delivery device 100, the drive assembly 236 is configured to engage the stopper 122 of the syringe 116, displace the syringe 116 such that the needle 120 pierces the skin of the patient, and displace the stopper 122 within the barrel 117 of the syringe 116 to deliver the medicament within the barrel 117. The drug delivery device 100 includes a storage position, a pre-use position, an actuation position, an injection position, and a post-use position, as described in International Patent Application Publication Nos. WO 2020/173991, WO 2020/173992, WO 2020/173993, WO 2020/173994, and WO 2020/173995.

Needle cover 124 is configured to shield the needle 120 of the syringe 116 from the patient when the device 100 is in the pre-use and the post-use positions. In particular, the needle cover 124 is moveable between a pre-use position, an actuation position, and a post-use positon, with a spring 125 biasing the needle cover 124 towards the pre-use position and the post-use position. In the pre-use position, the needle 120 may be positioned within the needle cover 124. In the actuation position, the needle cover 124 may be shifted proximally, thereby allowing the drive assembly 236 to be actuated. In the post-use position, the needle 120 may be again positioned within the needle cover 124. The spring 125 may be positioned between the needle cover 124 and the syringe holder 118, although other suitable arrangements may be utilized. The lever actuation member 130 is moveable between a locked position where movement of the drive assembly 236 is prevented and a released position where movement of the drive assembly 236 is allowed. More specifically, the lever actuation member 130 is rotatable about a rotation axis between the locked position and the released position. When the lever actuation member 130 is in the locked position, the lever actuation member 130 is engaged with the motor body 159 and the drive assembly 236 to prevent movement of the drive assembly 236.

When the lever actuation member 130 is in the released position, which may be achieved by pressing the needle cover 124 onto the patient's skin at the site of injection, shifting the needle cover proximally into the lower housing shell 112, the lever actuation member 130 is disengaged from the motor body 159 thereby allowing movement of the drive assembly 236 toward the syringe 116. Therefore, the drive assembly 236 may be configured to move the stopper 122 within the barrel 117 upon actuation of the drive assembly 236. The rotation axis of the lever actuation member 130 extends perpendicular to a longitudinal axis of the device 100, although other suitable arrangements may be utilized.

Referring again to FIGS. 1 and 2, the drive assembly 236 includes the drive member 156 and the plunger assembly 244. The drive member 156 may be a compression spring received within a drive opening 158 defined by the plunger body 150, although other suitable drive members may be utilized, including, but not limited to, compressed gas, an electric motor, hydraulic pressure, other types of springs, etc. The drive member 156 engages the plunger body 150 and the motor body 159 and biases the plunger body 150 in a direction extending from the second subassembly 140 toward the first subassembly 110. The plunger body 150 defines a lever opening that receives the lever actuation member 130 and defines the rotation axis of the lever actuation member 130. The lever actuation member 130 prevents movement of the plunger body 150 when the lever actuation member 130 is in the locked position through engagement of the lever actuation member 130 with the motor body 159. Upon rotation of the lever actuation member 130 from the locked position to the released position, the lever actuation member 130 is disengaged from the motor body 159 thereby allowing the drive member 156 to move the plunger body 150 and the plunger rod 152 toward the first subassembly 110. The plunger rod 152 and the drive member 156 are spaced from and parallel to each other and extend in a longitudinal direction of the device 100.

The drive assembly 236 further includes a spring guide member 154 secured to the upper housing shell 142 and received within the drive opening 158 of the plunger body 150. The drive member 156 is received by the spring guide member 154 such that the drive member 156 is positioned between the plunger body 150 and the spring guide member 154. The drive assembly 236 may also include a plunger rod cover that receives the plunger rod 152 of the plunger body 150. The plunger rod cover may be configured to guide insertion of the plunger rod 152 into the barrel 117 of the syringe 116 and engage the stopper 122 to dispense the medicament from the barrel 117 of the syringe 116. The plunger rod cover and the plunger rod 152 may be formed integrally or formed as separate components. The plunger rod 152 may have a proximal end 238 and a distal end 240, the plunger rod 152 comprising a clip 242 at the proximal end 238 thereof, wherein the clip 242 may be configured to be received within the opening 254 of the plunger body head 252 and to limit axial displacement between the plunger rod 152 and the plunger body 150, and to limit radial deflection of the plunger rod 152.

The plunger body 150 of the drive assembly 236 may also include an audio indicator member 160 configured to provide an audible indication to a user when the device 100 transitions to the post-use position. The audio indicator member 160 may be configured to engage one or more ribs of the cassette body 128 when the device 100 is in the injection position thereby deflecting the audio indicator member 160. When the drug delivery device 100 transitions from the injection position to the post-use position, the audio indicator member 160 may disengage from the rib(s) of the cassette body 128 and contact the lower housing shell 112 to provide an audible click, although the audio indicator member 160 could also contact other suitable portions of the device 100 to provide the audible indicator.

Referring to FIGS. 1-9, shown is a non-limiting embodiment of the drug delivery device 100 comprising the cap 114. The drug delivery device may comprise the housing 162 having a first end 164 and a second end 166, wherein the first end 164 comprises a distal region 170 defining a perimeter of the housing 162 and an adjacent proximal region 172 defining the perimeter of the housing 162, wherein in a first region 174, housing first end 164 is configured to engage a clip 173 from the cap 114. The distal region 170 comprises a raised plateau region 176 relative to an adjacent region 178 of the distal region 170 and the proximal region 172 comprises a recess 180 adjacent to the plateau region 176. The cap 114 may comprise a distal end 182 and a proximal end 184, wherein the proximal end 184 of the cap 114 may be engageable with the first end 164 of the housing 162, wherein the cap 114 may comprise a wall 186 defining a perimeter and the clip 173 protruding from the wall 186 at the proximal end 184 of the cap 114. When the housing 162 and the cap 114 are engaged, the wall 186 of the cap 114 may be in contact with the plateau region 176 of the housing 162, and the clip 173 may reside in the recess 180 of the housing 162.

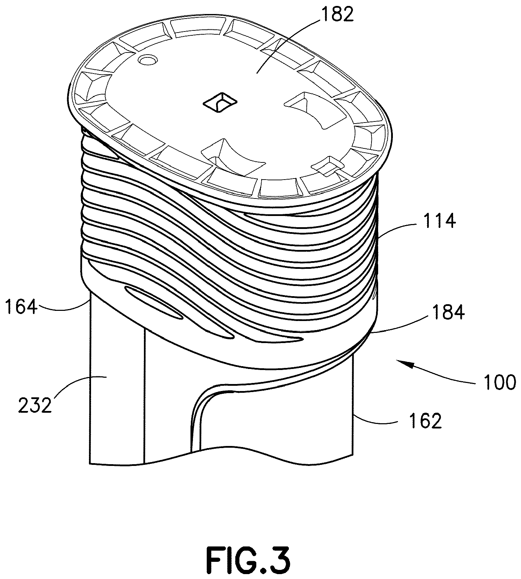

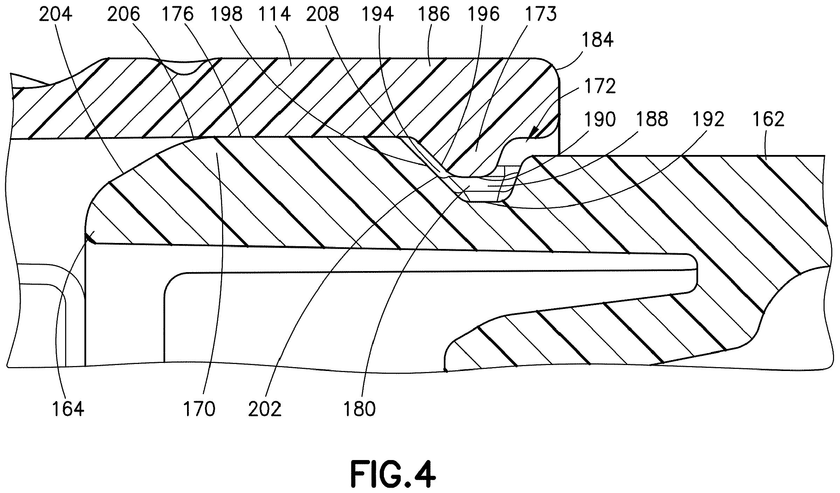

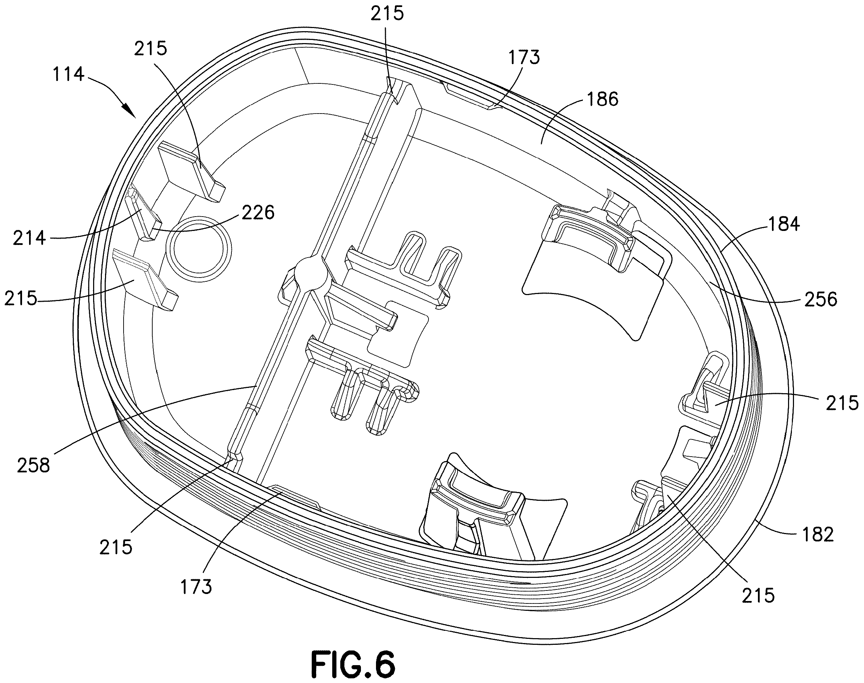

Referring to FIG. 3, the drug delivery device 100 comprising the cap 114 is shown according to some non-limiting embodiments. FIG. 3 shows the cap 114 engaged with the first end 164 (e.g., distal end) of the housing 162. The proximal end 184 of the cap 114 may engage with the first end 164 of the housing 162. The cap 114 may engage with an outside surface 232 of the housing 162. For example, an inside surface 256 (*see* FIG. 6) of the cap 114 may engage with the outside surface 232 of the housing 162. The proximal end 184 of the cap 114 may be reversibly engageable with the first end 164 of the housing 162, such that the cap may be removed and engaged with the housing 162 by a user as desired. FIGS. 4-9 show a non-limiting embodiment of the arrangement by which the housing 162 engages the cap 114.

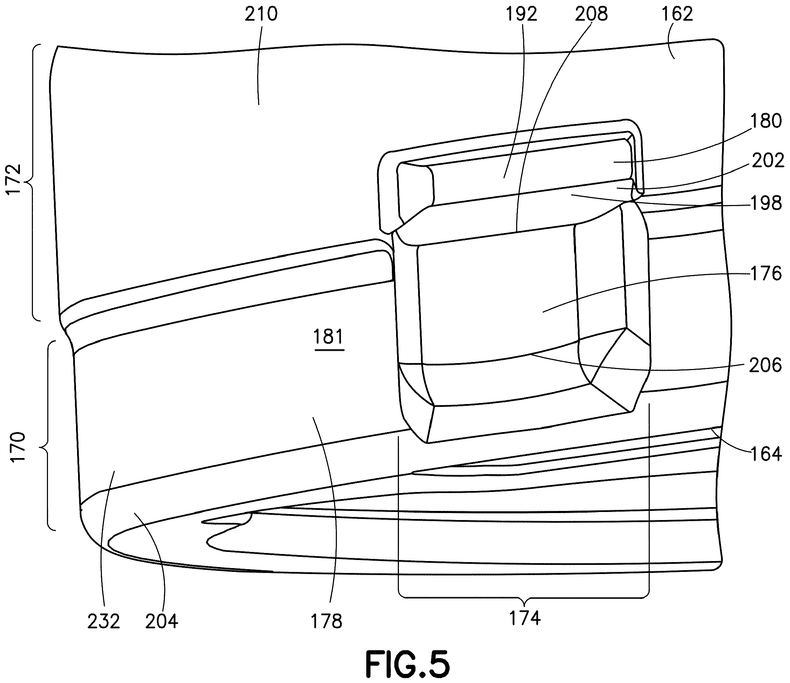

Referring to FIGS. 4-6, a non-limiting embodiment of the housing 162 engaging the cap 114 in the first region 174 of the drug delivery device 100 is shown. As shown in FIG. 5, the housing 162 of the drug delivery device 100 may comprise the distal region 170 defining a perimeter of the housing 162 and an adjacent proximal region 172 defining the perimeter of the housing 162. The first region 174 of the housing 162 may be configured to engage the clip 173 of the cap 114 (*see* FIG. 4) when the cap 114 and the housing 162 are engaged. In the first region 174, the distal region 170 comprises the raised plateau region 176, raised relative to an adjacent region 178 of the distal region 170. Raised plateau region 176 may define a larger perimeter of housing 162 than one or more remaining portions of first end 164, as shown in FIG. 4.

With continued reference to FIG. 5, in some regions of the first end 164 of the housing 162, the adjacent region 178 of the distal region 170 may be recessed 181 relative to an adjacent region 210 of the proximal region 172. The adjacent region 178 of the distal region 170 may be recessed 181 relative to the adjacent region 210 of the proximal region 172 around the entire perimeter of the housing 162 except in the first region(s) 174 and second region(s) 212 as described herein. The adjacent region 178 of the distal region 170 being recessed 181 relative to the adjacent region 210 of the proximal region 172 around the entire perimeter of the housing 162 except in the first region(s) 174 and second region(s) 212 may reduce the contact resistance between the cap 114 and the housing 162 when being disengaged from one another in order to keep the opening force required by a user to purposefully disengage the cap 114 from the housing 162 (e.g., during use of the drug delivery device 100) at a reasonably low level. The perimeter of the proximal region 172 may be larger than the perimeter of the distal region 170 except in the first region(s) 174 and second region(s) 212.

The first end 164 of the housing 162 may be chamfered and/or radiused 204. The chamfered and/or radiused 204 first end 164 may be positioned at a distal end 206 of the plateau region 176. As the first end 164 may be chamfered and/or radiused 204, the perimeter of the housing 162 at the first end 164 may become increasingly smaller distally along the length of the chamfer and/or radius 204.

The plateau region 176 in the distal region 170 in the first region 174 may be shaped as a plateau having a relatively flat top surface raised above the adjacent region 178 of the distal region 170. The entire surface of the plateau region 176 may be substantially perpendicular (within 5° of perpendicular) or perpendicular to the first end (e.g., a plane defining the first end). The entire surface of the plateau region 176 may be substantially parallel (within 5° of parallel) or parallel to the wall 186 of the cap 114 when the cap 114 engages the housing 162. The plateau region 176 may have the distal end 206 and a proximal end 208.

The recess 180 in the proximal region 172 in the first region 174 may be positioned adjacent the plateau region 176. The recess 180 may be shaped as a valley having a base 192 below an adjacent region of the proximal region 172. The recess 180 may have a distal end 198 and a proximal end. The distal end 198 of the recess may be chamfered 202, extending upward from the base 192 to the proximal end 208 of the plateau region 176.

Referring to FIG. 6, the cap 114 is shown according to some non-limiting embodiments. The cap 114 may include the distal end 182 and the proximal end 184, and the proximal end 184 of the cap 114 may be engageable with the first end 164 of the housing 162. The cap 114 may comprise the wall 186 defining the perimeter of the cap 114 and extending between the distal end 182 and the proximal end 184. The cap 114 may comprise a clip 173 protruding from the wall 186 at the proximal end 184 of the cap 114. The cap 114 may comprises a plurality of clips 173, such as two clips as shown in FIG. 6. The cap 114 may comprise the same number of clips 173 as the number of first regions 174 of the housing 162. The housing 162 may comprise a plurality of first regions 174, each configured to engage the clips 173 from the cap 114.

Referring to FIGS. 4-6, the cap 114 may be engaged with the housing 162. FIG. 4 shows a non-limiting embodiment of the cap 114 engaged with the housing 162, while FIGS. 5 and 6, respectively, show the housing 162 and cap 114 separately. In a pre-engagement position, the cap 114 and the housing 162 may be separated, as shown individually in FIGS. 5 and 6. To engage the cap 114 and the housing 162 the clips 173 of the cap 114 may be aligned with the first regions 174 of the housing 162. The clips 173 may slide over the chamfered and/or radiused 204 first end 164 of the housing 162, along the flat surface of plateau region 176, and into the recess 180, which secures the cap 114 to the housing 162. The cap 114 may be secured to the housing 162 when the clips 173 reside in the recess 180 of the housing 162.

FIG. 4 shows a non-limiting embodiment of the cap 114 secured to the housing 162, with the clip 173 residing in the recess 180. The wall 186 of the cap 114 in the region below the clip 173 may be in contact with the entire surface of the plateau region 176 when of the cap 114 is secured to the housing 162. The clip 173 residing in the recess 180 may define a gap 188 between a bottom 190 of the clip 173 and the base 192 of the recess 180. Including the gap 188 between a bottom 190 of the clip 173 and a base 192 of the recess 180 may make it such that the only contact between the cap 114 and the housing 162 in the first region 174 is at the plateau region 176. Additionally and/or alternative, the clip 173 residing in the recess 180 may define a gap 194 between a distal end 196 of the clip 173 and the distal end 198 of the recess 180. Including the gap 194 between the distal end 196 of the clip 173 and the distal end 198 of the recess 180 may make it such that the only contact between the cap 114 and the housing 162 in the first region 174 is at the plateau region 176. The distal end 196 of the clip 173 and the distal end 198 of the recess 180 may form substantially the same angle relative to the plateau region 176, such that their angles are within 5° of one another. For example, the distal end 196 of the clip 173 and the distal end 198 of the recess 180 may form the same angle relative to the plateau region 176. In this way, the distal end 196 of the clip 173 and the distal end 198 of the recess 180 may be substantially parallel or parallel with one another when the clip 173 resides in the recess 180.

The engagement of the cap 114 and the housing 162 in the first region 174 as shown and described in FIGS. 4-6 may provide good stabilization between the cap 114 and housing 162 to reduce the risk of the cap 114 inadvertently being dislodged from the housing 162, while keeping the opening force required by a user to purposefully disengage the cap 114 from the housing 162 (e.g., during use of the drug delivery device 100) at a reasonably low level.

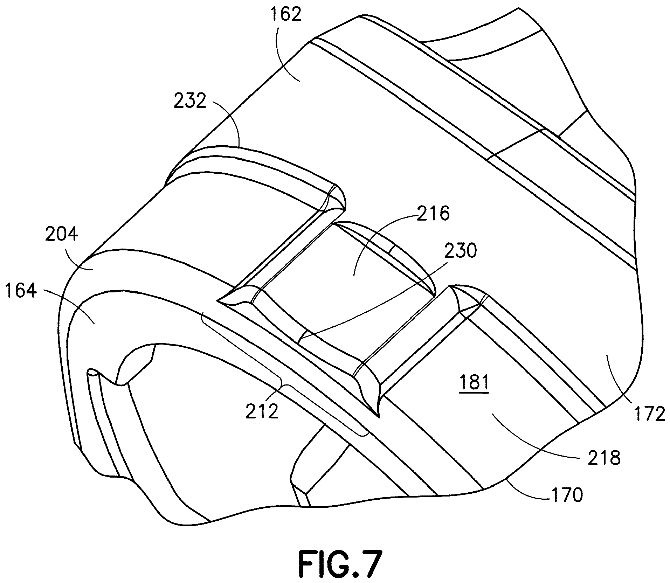

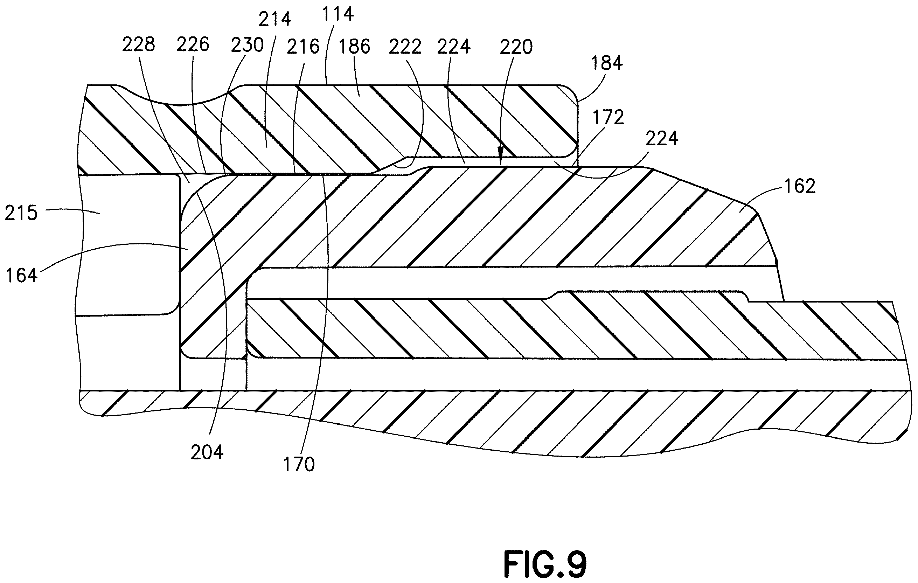

Referring to FIGS. 6-9, a non-limiting embodiment of the drug delivery device 100 engaging the cap 114 in the second region 212 of the drug delivery device 100 is shown. As shown in FIG. 7, the housing 162 of the drug delivery device 100 may comprise the second region 212 configured to engage a rib 214 from the cap 114. The housing 162 may comprise one or more second regions 212, each of the one or more configured to engage the ribs 214 from the cap 114. The housing 162 may comprise the same number of second regions 212 as the number of ribs 214 in the cap 114. The second region 212 of the housing 162 may be configured to engage the rib 214 of the cap 114 (*see* FIG. 9) when the cap 114 and the housing 162 are engaged.

Referring to FIG. 7, in the second region 212, the distal region 170 may comprise a second raised plateau region 216, raised relative to an adjacent region 218 of the distal region 170. The second plateau region 216 may have a proximal end and a distal end 230. The second plateau region 216 may have substantially the same shape as described in connection with the plateau region 176. The second region 212 may not include a recess 180 as described in connection with the first region 174.

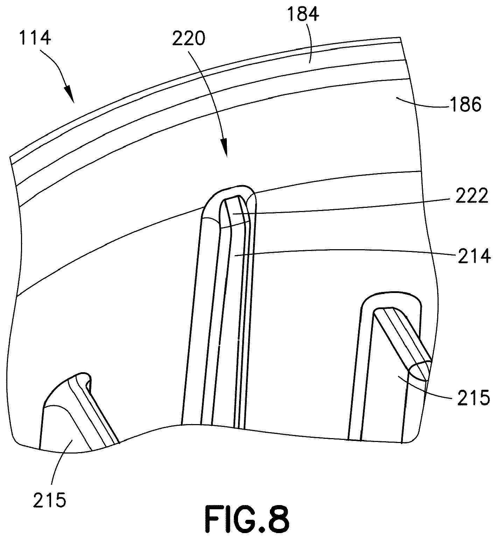

Referring to FIGS. 6 and 8, the rib 214 may protrude from the wall 186 of the cap 114 and may engage with the second plateau region 216 when the housing 162 and the cap 114 are engaged. The rib 214 may comprise a top 222, proximal end and a distal end 226. The cap 114 may comprise a gap 220 formed between the top 222 of the rib 214 and the proximal end 184 of the cap 114. For the rib 214, the distal end 226 may be shaped so as to define a gap 228 between the rib 214 and the chamfered and/or radiused 204 first end 164 of the housing 162 adjacent the distal end 230 of the second plateau region 216 when the housing 162 and the cap 114 are engaged. A rib 214 may be arranged substantially opposite (40-60% around the length of the perimeter of the cap 114) another rib 214 of the cap 114.

Referring to FIGS. 6 and 8, the cap 114 may comprise a support 215. The cap 114 may comprise a plurality of supports 215. The cap 114 may comprise a plurality of different arrangements of supports 215, such as first supports 215 and second supports 215 having a distinct design from the first supports 215. The cap 114 may comprise a plurality of first supports 215 and/or a plurality of second supports 215.

The first support 215 may protrude from the wall 186 of the cap 114 and may engage with the first end 164 when the housing 162 and the cap 114 are engaged. The top of the first support 215 may abut the first end 164 of the housing 162. A plurality of first supports 215 may be arranged proximate to one another around the perimeter of the cap 114, such as at least two first supports 215 within a same quadrant (25% of the length) of the perimeter. The rib 214 may be arranged between the pair of proximate first supports 21. A plurality of first supports 215 may be arranged substantially opposite (40-60% around the length of the perimeter of the cap 114) another pair of first supports 215, between which another rib 214 may be arranged.

The second support 215 may protrude from the wall 186 of the cap 114 and be formed by the abutment of the wall 186 with an inner wall 258 of the cap 114 spanning between two points of the wall 186 of the cap 114. The second support 215 may engage with the first end 164 when the housing 162 and the cap 114 are engaged. The top of the second support 215 may abut the first end 164 of the housing 162.

Referring to FIGS. 6-9, the cap 114 may be engaged with the housing 162. FIG. 9 shows a non-limiting embodiment of the cap 114 engaged with the housing 162 in the second region 212, while FIGS. 6-8 show the housing 162 or cap 114, separately. In a pre-engagement position, the cap 114 and the housing 162 may be separated, as shown individually in FIGS. 6-8. To engage the cap 114 and the housing 162, the ribs 214 of the cap 114 may be aligned with the second regions 212 of the housing 162. The ribs 214 may slide over the chamfered and/or radiused 204 first end 164 of the housing 162 and along the flat surface of second plateau region 216 until the clips 173 and the recesses 180 are engaged in the first regions 174, which secures the cap 114 to the housing 162.

FIG. 9 shows a non-limiting embodiment of the cap 114 secured to the housing 162, with the rib 214 engaging with the second plateau region 216, and the support 215 abutting the first end 164 of the housing 162. The rib 214 of the cap 114 may be in contact with the entire surface of the second plateau region 216 when of the cap 114 is secured to the housing 162. When the cap 114 is engaged with the housing 162, a gap 224 may be formed between the proximal region 172 of the housing 162 and the wall 186 of the cap 114, which may be formed due to the gap 220 between the top 222 of the rib 214 and the proximal end 184 of the cap 114. The proximal region 172 of the housing 162 and the wall 186 of the cap 114 may be substantially parallel with one another to form the gap 224. The gap 224 between the proximal region 172 of the housing 162 and the wall 186 of the cap 114 may be maintained around the perimeter of both the cap 114 and the housing 162 except in the region where the clip 173 engages the recess 180 in the first region 174.

With continued reference to FIG. 9, when the cap 114 is engaged with the housing 162, the gap 228 may be formed between the rib 214 and the chamfered and/or radiused 204 first end 164 of the housing 162 adjacent the distal end 230 of the second plateau region 216. This gap 228 may be formed due to the shape of the distal end 226 of the rib 214, such that it does not completely conform to the shape of the chamfered and/or radiused 204 first end 164 of the housing 162.

The rib 214 of the cap 114 may be in contact with the second plateau region 216 of the housing 162 across the entire surface of the second plateau region 216.

The engagement of the cap 114 and the housing 162 in the second region 212 as shown and described in FIGS. 6-9 may provide good stabilization between the cap 114 and the housing 162 to reduce the risk of the cap 114 inadvertently being dislodged from the housing 162, while keeping the opening force required by a user to purposefully disengage the cap 114 from the housing 162 (e.g., during use of the drug delivery device 100) at a reasonably low level.

Referring to FIGS. 1-9, the present invention is also directed to a method of manufacturing a drug delivery device, such as the drug delivery device 100 disclosed herein. The method may include engaging the housing 162 (as described herein) of the drug delivery device 100 with the cap 114 (as described herein). During engagement of the housing 162 and the cap 114, the first regions 174 of the housing 162 may be engaged with the clips 173 of the cap 114, and the second regions 212 of the housing 162 may be engaged with the ribs 214 of the cap 114.

With continued reference to FIGS. 1-9, the present invention is also directed to a method of injecting a drug from a drug delivery device, such as the drug delivery device 100 disclosed herein. The method may include disengaging the cap 114 from the housing 162 of the drug delivery device 100 comprising a drug and injecting the needle 120 contained in the first end 164 of the housing 162 into a patient to inject the drug into the patient. During disengagement of the housing 162 and the cap 114, the first regions 174 of the housing 162 may be disengaged with the clips 173 of the cap 114, and the second regions 212 of the housing 162 may be disengaged with the ribs 214 of the cap 114.

Although the present disclosure has been described in detail for the purpose of illustration based on what is currently considered to be the most practical and preferred embodiments, it is to be understood that such detail is solely for that purpose and that the present disclosure is not limited to the disclosed embodiments, but, on the contrary, is intended to cover modifications and equivalent arrangements that are within the spirit and scope of the appended claims. For example, it is to be understood that the present disclosure contemplates that, to the extent possible, one or more features of any embodiment can be combined with one or more features of any other embodiment.

## Claims

1. A drug delivery device, comprising:
a housing having a first end and a second end, wherein the first end comprises a distal region defining a perimeter of the housing and an adjacent proximal region defining the perimeter of the housing, wherein in a first region configured to engage a clip from a cap, the distal region comprises a raised plateau region relative to an adjacent region of the distal region and the proximal region comprises a recess adjacent to the plateau region; and
the cap comprising a distal end and a proximal end, wherein the proximal end of the cap is engageable with the first end of the housing, wherein the cap comprises a wall defining a perimeter and the clip protruding from the wall at the proximal end of the cap,
wherein when the housing and the cap are engaged, the wall of the cap is in contact with the plateau region of the housing, and the clip resides in the recess of the housing.

2. The drug delivery device of claim 1, wherein the housing comprises a plurality of first regions, each configured to engage a clip from the cap.

3. The drug delivery device of claim 1 or claim 2, wherein an arrangement of the clip residing in the recess of the housing defines a gap between a bottom of the clip and a base of the recess and/or defines a gap between a distal end of the clip and a distal end of the recess.

4. The drug delivery device of claim 3, wherein the distal end of the clip and the distal end of the recess form substantially the same angle relative to the plateau region.

5. The drug delivery device of claim 3, wherein the distal end of the recess is chamfered.

6. The drug delivery device of any of claims 1-5, wherein the housing comprises a chamfered distal end adjacent a distal end of the plateau region.

7. The drug delivery device of any of claims 1-6, wherein the adjacent region of the distal region is recessed relative to an adjacent region of the proximal region.

8. The drug delivery device of any of claims 1-7, wherein the housing further comprises a second region configured to engage a rib from the cap, wherein in the second region, the distal region comprises a second raised plateau region relative to an adjacent region of the distal region.

9. The drug delivery device of claim 8, wherein the cap comprises the rib protruding from the wall of the cap, wherein, when the housing and the cap are engaged, the rib of the cap is in contact with the second plateau region of the housing.

10. The drug delivery device of claim 9, wherein the cap comprises a gap between a top of the rib and the proximal end of the cap such that a gap is formed between the proximal region of the housing and the wall of the cap when the housing and the cap are engaged.

11. The drug delivery device of claim 8, wherein the housing comprises a plurality of second regions, each configured to engage a rib from the cap.

12. The drug delivery device of claim 9, wherein the housing comprises a chamfered distal end adjacent a distal end of the second plateau region, wherein the rib comprises a distal end shaped so as to define a gap between the rib and the chamfered distal end adjacent the distal end of the second plateau region when the housing and the cap are engaged.

13. The drug delivery device of any of claims 1-12, wherein the cap engages an outside surface of the housing.

14. The drug delivery device of claim 8, wherein an entire surface of the plateau region and/or second plateau region are substantially parallel to the wall of the cap when the cap and the housing are engaged.

15. The drug delivery device of claim 14, wherein the wall of the cap is in contact with the plateau region of the housing across the entire surface of the plateau region, and/or the rib of the cap is in contact with the second plateau region of the housing across the entire surface of the second plateau region.
